# EUROPEAN PATENT SPECIFICATION

(11) **EP 3 329 957 B1**
(45) Date of publication and mention of the grant of the patent: **21.08.2024**
(21) Application number: 16201769.3
(22) Date of filing: 01.12.2016
(51) Int. Cl.: A61M 25/00, A61M 39/26, A61M 25/06, A61M 39/06

(54) **INTRAVENOUS CATHETER WITH BLOOD STOP FEATURE**
INTRAVENÖSER KATHETER MIT BLUTSTOPPMERKMAL
CATHÉTER INTRAVEINEUX AVEC FONCTION ANTI-HÉMORRAGIQUE

(43) Date of publication of application: 06.06.2018
(73) Proprietor: Delta Med S.p.A., 46019 Viadana (IT)
(72) Inventor: Ronzoni, Mattia, 24030 Brembate di Spora (BG) (IT)
(74) Representative: GCA S.R.L.

(56) References cited:
- WO-A1-00/16834
- US-A- 4 917 668
- US-A- 6 162 201
- US-A1- 2007 083 157

## Description

### 1. Technical field

The present invention generally relates to the field of medical needle assemblies, and more particularly to intravenous catheters which prevent blood spillage.

### 2. The prior art

In the field of intravenous catheters, the clinical indications for venipuncture prescribe to apply a pressure on the patient's vein, e.g. using a finger, in order to avoid blood spillage during catheter insertion. During this procedure, oftentimes leakages happen, exposing clinical operators to the risks connected with the contact with potentially infected blood. In order to reduce this risk, some companies have developed intravenous (IV) catheters provided with different mechanisms designed to prevent blood spillage during catheter insertion. These mechanisms are commonly referred as "blood stop" or "blood control" features. In particular, the focus is currently on so-called "passive" devices, in which blood-control systems are automated and no action is required at the operator side. For example:
US 2009/281481 A1 discloses an apparatus to control blood flow through an intravenous catheter that may include a resilient outer shell and an inner valve portion formed therein. Therein, the inner valve portion may be configured to open upon compressing the resilient outer shell.

Compressing the resilient outer shell by, for example, inserting a Luer device into a catheter adapter containing the resilient shell, may cause the mating portions to misalign, thereby creating a fluid path. However, this requires compression of the outer shell. Further, this requires a complex valve construction. Lastly, this construction is not able to apply (relatively) high closing forces on the valve.

US 2014/276462 A1 discloses a multiple-use intravenous (IV) catheter assembly septum and septum actuator, wherein the IV catheter assembly has a stationary septum actuator and a blood control septum, wherein the blood control septum is configured to slide within a catheter adapter of the IV catheter assembly between a compressed state and an uncompressed state. When in the compressed state, a slit of the blood control septum is opened and the septum comprises stored compressive potential energy. When the septum is released from the compressed state, the stored compressive potential energy is released and the blood control septum is restored to its original shape, thereby closing the septum' s slit. However, as the septum is deformed and stores potential energy, it is also subject to external factors as temperature, modification during sterilization, aging and fatigue.

US 2015/0151089 A1 discloses a catheter assembly including a catheter hub and an introducer needle. Therein, the introducer needle extends through the catheter hub and through a catheter tube so as to assist placement of the catheter tube into a patient's blood vessel. Blood flashback into the catheter tube and/or catheter hub can indicate when the catheter tube is properly positioned within the blood vessel. After proper catheter tube placement is confirmed through blood flashback, the introducer needle is withdrawn. A septum blocks flashback blood from flowing proximally out of the catheter hub. Air vents enable air within the hub to vent when flashback blood enters the catheter hub. A porous hydrophobic material covers the air vents. The hydrophobic material allows the air to flow therethrough and through the vents, but repels blood, blocking blood from flowing through the air vents. However, this requires a complex construction of the blood stop feature which results in high manufacturing costs.

Furthermore, there are also active devices known in the prior art which require active human intervention. For example, Medikit's Supercath^{™} 5 discloses a check valve and a plunger in order to provide an IV catheter with a blood stop feature. However, this requires two distinct parts for the blood stop feature to be realized. Further, this device is an active device, needing a finger push of the operator in order to enable the blood stop feature.

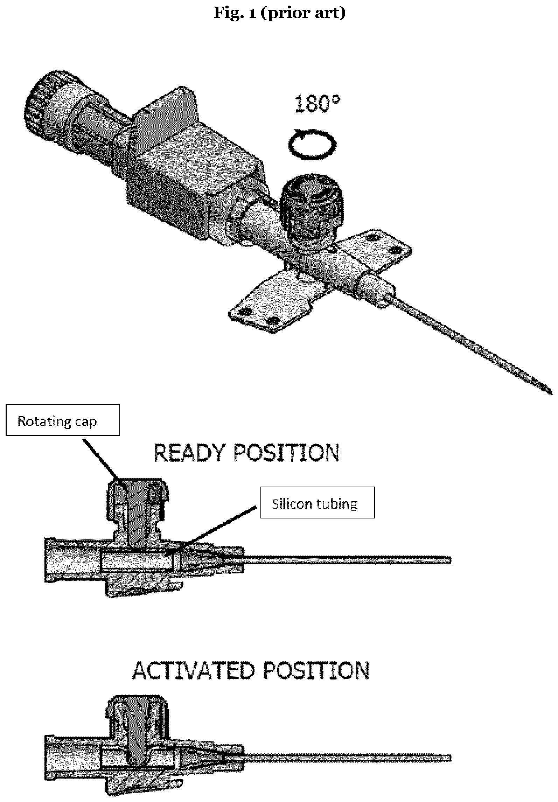

Italian patent MO20134A000059 of applicant discloses an active blood control system. Therein, the operator has to manually activate the blood stop feature by rotating a plastic cap around 180 degrees which causes a piston to move downwards and apply a pressure on a silicon tube such that it closes the passageway for fluids (see Fig. 1). Therein, the device has two positions, a "ready position" in which the piston does not affect the silicon tubing, and an "activated position" in which the piston compresses the tube such that it closes the passageway for fluids. Rotating the cap causes the device to transition between the ready position and the activated position. However, this requires the operator to intervene manually. Further, this requires the device to be considerably larger in size. Moreover, especially in countries in which nurses are not well trained or in those wards in which operators are constantly under pressure (e.g. emergency room departments), such a manual blood stop feature has a wide variety of disadvantages.

In US 2007083157 an IV catheter with in-line valve and methods related thereto are disclosed. The catheter includes an housing, a tubular member, a seal member, a compression member and a security mechanism. The housing includes a proximal and a distal portion and a chamber that extends between the proximal and distal housing. The tubular member is coupled to the housing distal portion so it is fluidly coupled to the chamber. The seal member is disposed within the chamber and the security mechanism secures the seal member distal end to the housing proximal portion so the seal member is sealingly and compressibly retained between a chamber proximal end and the securing mechanism. Such a seal member also forms a septum, which is compressed by the compression member to prevent seepage upon removal of a stylet.

In WO0016834 an hemodialysis access apparatus is disclosed. The apparatus is an implanted single or dual. Lumen device for repeated accessing of a vessel within a body, especially for hemodialysis, plasmapheresis, and other fluid exchange therapy treatments. The device has no septum for sealing but has a resilient material to form a seal, a smooth streamlined flow path with a low flow resistance, substantially no stagnation points, such that the device is easily and completely flushed. Optionally, a gradually changing direction of flow path without introducing flow resistance or stagnation points with a change in direction of up to 20 degrees with a minimum bend radius of four times the lumen diameter. A positive locking mechanism that accepts and retains a matching needle apparatus. The apparatus is joined to a catheter, in most cases, such that fluids can be extracted for or injected into the vessel or vessels to be accessed.

In US 6162201 an internal urinary catheter is disclosed. The urinary catheter includes a cannula having urine passage, a urine inlet and a urine outlet and a valve positioned in said urine passage between said inlet and said outlet and having a valve passage having at least a portion which is moveably positioned between a substantially blocked position and a flow position, and structure for moving said portion. Said structure is biased toward a blocking position wherein said portion is in said substantially blocked position and flow through said valve means is substantially blocked. Said structure for moving is moveable to a position at least partially moving said portion toward said flow position wherein flow through said valve means is allowed.

In US 4917668 a valve for permanent venous cannulae or for catheter insertion means is disclosed. In the valve, a housing having a channel is provided with a slotted, elastomeric valve body dividing the channel. The slotted valve body is biased in the closed position by a metal spring. If the valve body's material loses resiliency, the spring guarantees that the valve takes its closed position, even if the valve body is kept in the opened state for a considerable span of time by a steel cannula inserted through the valve body.

It is therefore the technical problem underlying the present invention to provide an IV catheter device with a passive blood stop feature which is simple to construct, can apply high closing forces and which is more resistant to surrounding factors like temperature, modification during sterilization, aging and fatigue and thereby at least partly overcomes the above explained disadvantages of the prior art.

### 3. Summary of the invention

The invention is defined by the independent claim.

Advantageous modifications of embodiments of the invention are defined in the dependent claims.

In the embodiment of claim 1, an intravenous catheter device comprises a catheter hub comprising an inner cavity, a flexible tube arranged within the inner cavity to provide a closeable passageway for fluid through the catheter hub, and a flexible spring clip which is movable between a deactivated position and an activated position. The spring clip compresses the flexible tube in the activated position to close the passageway. The spring clip is moveable from the activated position to the deactivated position when a fluid connector is inserted into the catheter hub.

From a manufacturing point of view, the IV catheter device is easier to manufacture because the mechanism is based on a spring clip which is cheaper to manufacture and easier to assemble in an automatic process. Further, silicon valves are manufactured by injection molding, which is a costly process. Furthermore, silicon valves are less easy to handle during the assembling process.

According to the invention, no custom-made silicon valve needs to be used, but just a common silicon tubing. Therein, the silicon tubing may be used for an additional purpose as well, which is to implement the blood stop feature. Therein, the only feature that needs to be added to create a blood stop feature is a spring clip. In this way, another object of the invention may be achieved, in that the catheter hub does not have to be much larger than the ones without blood-stop feature. In the prior art, there are products that are two or three times bigger in dimensions in order to contain a blood stop feature.

In one aspect of the invention, the spring clip is moveable without human intervention, i.e. without the operator having to touch the spring clip and/or manually activate the spring clip. In other words, the spring clip closes the fluid passage fully automatically during normal use of the device. Accordingly, the risk of contact with potentially infected blood for the clinical operators may be reduced, as fluids can only pass through the passageway of the IV catheter when the spring clip is in deactivated position. When the transition from the deactivated state to the activated state of the spring clip is done automatically, the risk of blood spillage can be further reduced. This is particularly helpful in regions where nurses are less trained and/or regions with a high ration of patients having contaminated blood.

Additionally, or alternatively the spring clip is moveable from the deactivated position into to the activated position when the fluid connector is retracted from the catheter hub. This way, it can be ensured that the IV catheter device is automatically "closed" (the device is an activated position) when the fluid connector is retracted from the device. Accordingly, the risk of contact with potentially infected blood for the clinical operators may be reduced, as fluids can only pass through the passageway of the IV catheter when the spring clip is in deactivated position. This may be particularly helpful in the case of blood drawing or blood sampling. Accordingly, the blood stop feature according to an embodiment of the invention may function more than once. Meaning that one can insert the catheter, draw back the needle, connect the infusion line and the disconnect it and reconnect theoretically infinite times. This cannot be assured by the prior art blood stop features relying in silicon valves as these valves are subject to fatigue and shape-memory.

According to the invention, the inner cavity of the catheter hub comprises two retaining cavities in which at least a part of the spring clip is arranged, wherein each retaining cavity has a trapezoid form at the distal end. This way, a simple construction of the inner cavity is enabled. Further, the fabrication of such an inner cavity is easy and cheap. This is particularly advantageous, especially for regions where elevated prices for additional blood stop features for IV catheters are not affordable. Furthermore, the trapezoid form of the inner cavity enables the spring clip to move automatically as the inner cavity is not anchoring the spring clip irreversibly, therefore leading to a passive device.

In addition to the foregoing, in an embodiment at least a part of the spring clip is moved into the distal portion of each of the retaining cavities when the spring clip is moved from the activated position into the deactivated position, causing the spring clip to no longer compress the flexible tube. Accordingly, the spring clip moves into the retaining cavities, wherein it is caused to no longer compress the flexible tube, therefore transitioning from an activated position into a deactivated position. Further, the simple construction of the device may allow for lower fabrication costs. This is particularly helpful for regions where nurses are less trained and/or regions with a high ration of patients having contaminated blood and in regions where expensive catheter devices are not affordable.

In an embodiment of the invention, the spring clip comprises two opposing arms. A spring clip comprising two opposing arms is able to apply a higher closing force onto the flexible tube than only a single arm could do.

In addition to the foregoing, in an embodiment the two opposing arms comprise each a segment formed in direction towards the tube, and wherein each of the two segments compress the tube in the activated position. This way, even higher closing forces can be applied onto the flexible tube as the applied pressure is more punctual.

Further, in addition to the foregoing, in an embodiment the two segments compressing the tube in the activated position further comprise a rounded end in order to prevent the tube from taking damage by the two compressing segments of the two opposing arms. Accordingly, damage to the flexible tube can be avoided. By using rounded ends for the segments compressing the tube, even relatively high closing forces can be applied to the tube multiple times without damaging the tube. Therefore, the durability of the device can be enhanced.

In an aspect of the invention, the two opposing arms of the spring clip are connected. By connecting the two opposing arms of the flexible spring clip, a higher closing force can be applied as would be possible by two arms that apply the pressure separately.

According to another aspect of the invention, the spring clip is made of a resilient material, such as metal or plastic material, wherein the spring clip has its natural shape in the activated position and wherein the spring clip is urged to the outside in the deactivated position. This way, a passive device with an easy construction is provided, as only the way of transition from the activated position into the deactivated position needs to be constructed, as the reverse transition is automated due to span energy saved in the flexible spring clip.

The spring clip may be fabricated of a single piece. Accordingly, an easy fabrication is enabled by the simple construction of the device. Therefore, the device can be fabricated in a cheap way, which is particularly advantageous for regions where nurses are not well trained and where expensive catheter device are not affordable.

Furthermore, the flexible tube may be made of silicon. Silicon is already present in most IV catheters. Therefore, only light changes to IV catheters without blood stop feature need to be done in order to construct catheters having a blood stop feature. In consequence, the fabrication costs of IV catheters with a passive blood stop feature can be lowered.

According to yet another aspect of the invention, the spring clip does not extend beyond the inner cavity of the catheter hub. This way, the IV catheter device may have the same dimensions as IV catheter devices without (active or passive) blood stop feature.

Preferably, the spring clip is in the activated position before using the device for a first time. This way, an operator may be protected of initial blood spillage while e.g. blood drawing or sampling. Further, the spring clip being in the activated position before using the device for a first time may be due to the needle being inserted in a support that may have a Luer cone shape.

The present invention also provides a flexible spring clip for use in an intravenous catheter device in accordance to the embodiments explained above.

### 4. Short description of the drawings

In the following detailed description, presently preferred embodiments of the invention are further described with reference to the following figures:
- Fig. 1:: An active intravenous catheter device according to the prior art;
- Fig. 2:: An intravenous catheter device, wherein a flexible spring clip is in activated position according to an embodiment of the invention;
- Fig. 3:: The intravenous catheter device of Fig. 2, wherein the flexible spring clip is in transition from the activated position towards a deactivated position according to an embodiment of the invention;
- Figs. 4a-b:: A flexible spring clip of the intravenous catheter device according to an embodiment of the invention.

### 5. Detailed description of preferred embodiments

In the following, presently preferred embodiments of the invention are described with respect to an IV catheter having a passive blood stop feature for a medical needle assembly which serves for reducing the risk of contact with potentially infected blood for the operator. The IV catheter device may be used for various applications, wherein exemplary applications may include without limitation blood draw, blood sample and/or infusion.

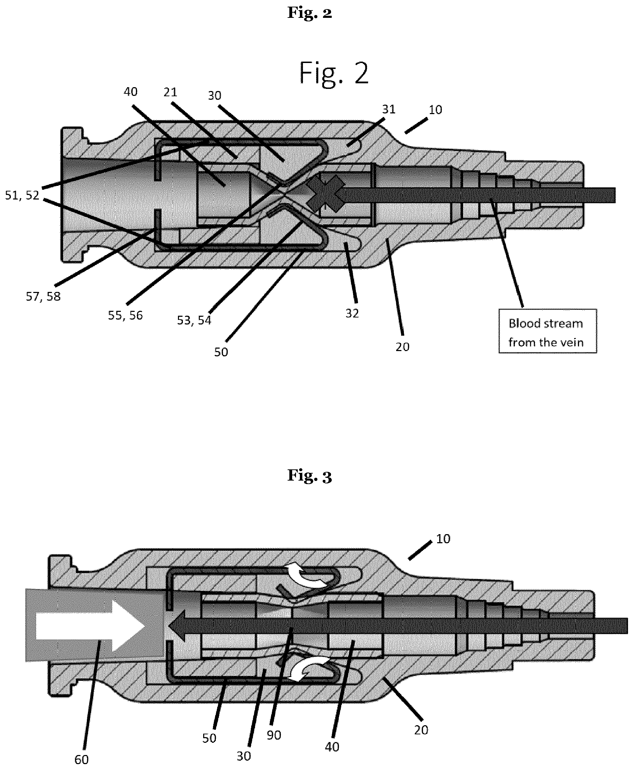

Fig. 2 depicts an IV catheter device 10, wherein a flexible spring clip 50 is in activated position 70 according to an embodiment of the invention. Therein, a catheter hub 20 is shown. On the inner side of the catheter hub 20, there is an inner cavity 30, a silicon tube 40, and a metal spring 50. While the invention will be explained in connection with a metal spring 50, it will be appreciated that any other resilient material, such as plastic material, is also suitable for the present invention.

As can be seen, the inner cavity 30 comprises two retaining cavities 31, 32. The metal spring clip 50 has two opposing arms 51, 52 which each have segments 53, 54 formed towards the tube 40. Further, the metal spring clip 50 comprises rounded ends 55, 56 at each of the two opposing arms 51, 52. At the proximal end of the spring clip 50, there are two retaining portions 57, 58. Within the retaining cavities 31, 32 there are provided respective wall segments 21 which prevent the spring clip 50 from being moved indefinitely into distal direction. In the embodiment of Fig. 2, this is achieved in that proximal ends of the wall segments 21 provide a barrier for the retaining portions 57, 58. However, preventing the spring clip 50 from being moved indefinitely into distal direction may also be achieved without the wall segments 21. Therein, the spring clip 50 may be stopped by means of the two retaining cavities 31, 32.

In Fig. 2, the metal spring clip 50 of the IV catheter device is in an activated position 70, meaning that the metal spring 50 is in its natural shape in which it clamps the flexible tube 40 such that the passageway 90 through the tube 40 is closed, thereby hindering any fluid from moving through the passageway 90. This way, on the one hand, a blood stream from the vein is hindered to pass from the vein of a patient through the catheter hub 20, and on the other hand, fluids are hindered to flow through the catheter hub 20 into the blood vessel of a patient. This is indicated by the cross in the flexible tube 40 in Fig. 2. Fig. 3 depicts the IV catheter device 10, wherein the metal spring clip 50 is in transition from the activated position 70 towards a deactivated position 80. Therein, the structure of the device 10 corresponds to the structure of the IV catheter device 10 of Fig. 2. However, in comparison to Fig. 2 there is an additional fluid connector, such as an infusion line plug, which in this example is a Luer cone 60. The Luer cone 60 is inserted into the catheter hub 20 from the proximal side.

The insertion of the Luer cone 60 causes the distal ends of the metal spring 50 to move into distal direction (co-directional to the Luer cone 60). As the spring clip 50 moves into distal direction, the two segments 53, 54 formed towards the tube 40 of the spring clip start to touch the retaining cavities 31, 32 of the inner cavity 30 of the catheter hub 20. When the spring clip 50 is now moved further into distal direction, e.g. by the Luer cone 60, the two segments 53, 54 formed towards the tube 40 start to deform which causes the distance between each of the two opposing arms 51, 52 and their corresponding rounded ends 55, 56 to shrink. In consequence of the shrinking distance between the two opposing arms 51, 52 and their corresponding rounded ends 55, 56 the clamped silicon tube 40 gains space to deform into its natural form, causing the passageway 90 to open and giving fluids the possibility of flowing through the passageway 90. The further the metal spring 50 is pushed into distal direction, the more the spring clip 50 deformes into the form of the two retaining cavities 31, 32 of the inner cavity 30 of the catheter hub 20, which causes the flexible tube 40 to regain its original form and subsequently opening the clamped passageway 90.

During this process the energy of pushing the spring clip 50 into distal direction, e.g. by the Luer cone 60, is transformed into span energy in the spring clip 50. Thus, it will be understood that when the Luer cone 60 is retracted in proximal direction from the catheter hub 20, the saved span energy in the spring clip 50 preferably causes the spring clip 50 to flip back out of the retaining cavities 31, 32 of the inner cavity 30, which will lead to a transition of the spring clip 50 from the deactivated position 80 back into the activated position 70, as depicted in Fig. 2.

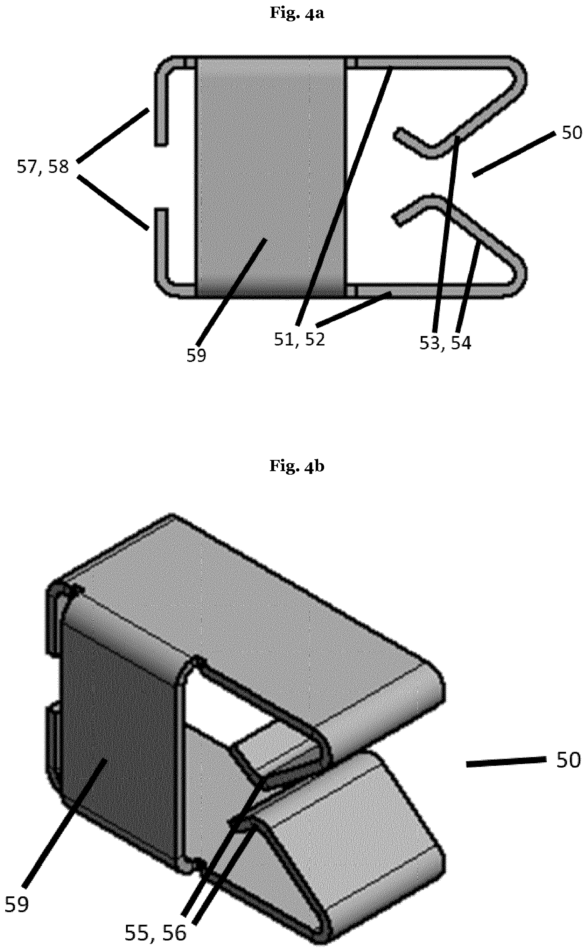

Figs. 4a and 4b depict a flexible spring clip 50 of the intravenous catheter device 10 in a side view and a perspective view, respectively. As can be seen, the two arms 51, 52 are preferably connected by an element 59, thereby ensuring a parallel movement of both parts of the spring clip 50. Preferably, the spring clip 50 comprises only one connecting element 59, which saves material and simplifies the manufacturing process.

Each of the two opposing two arms 51, 52 has a segment which is formed towards the inner side 53, 54 of the spring clip 50. Therein, at the end of each of these segments 53, 54 the spring clip 50 comprises rounded ends 55, 56.

Further, the spring clip 50 is in this preferred embodiment fabricated of a single piece of sheet material.

### Reference signs:

| | |
|---|---|
| IV catheter device | 10 |
| catheter hub | 20 |
| wall segments | 21 |
| inner cavity | 30 |
| retaining cavities | 31, 32 |
| silicon tube | 40 |
| metal spring | 50 |
| opposing arms | 51, 52 |
| segments | 53, 54 |
| rounded ends | 55, 56 |
| retaining portions | 57, 58 |
| connection | 59 |
| Luer cone | 60 |
| activated position | 70 |
| deactivated position | 80 |
| passageway | 90 |

## Claims

1. An intravenous catheter device (10), comprising:
a. a catheter hub (20) comprising an inner cavity (30) ;
b. a flexible tube (40) arranged within the inner cavity (30) to provide a closeable passageway (90) for fluid through the catheter hub (20); and
c. a flexible spring clip (50) which is movable between a deactivated position (80) and an activated position (70), wherein the spring clip (50) compresses the flexible tube (40) in the activated position (70) to close the passageway (90) ;
d. the spring clip (50) being moveable from the activated position (70) to the deactivated position (80) when a fluid connector (60) is inserted into the catheter hub (20),
**characterized in that** the inner cavity (30) of the catheter hub (20) comprises two retaining cavities (31, 32) in which at least a part of the spring clip (50) is arranged, wherein each retaining cavity (31, 32) has a trapezoid form at the distal end.

2. The device of claim 1, wherein the spring clip (50) is moveable without the operator having to touch the spring clip.

3. The device of any of the preceding claims, wherein the spring clip (50) is moveable from the deactivated position (80) into to the activated position (70) when the fluid connector (60) is retracted from the catheter hub (20).

4. The device of the preceding claim, wherein at least a part of the spring clip (50) is moved into the distal portion of each of the retaining cavities (31, 32) when the spring clip (50) is moved from the activated position (70) into the deactivated position (80), causing the spring clip (50) to no longer compress the flexible tube (40).

5. The device of any of the preceding claims, wherein the spring clip (50) comprises two opposing arms (51, 52).

6. The device of the preceding claim, wherein the two opposing arms (51, 52) comprise each a segment (53, 54) formed in direction towards the tube (40), and wherein each of the two segments (53, 54) compresses the tube (40) in the activated position (70).

7. The device of claim 6, wherein the two segments (53, 54) further comprise a rounded end (55, 56) to prevent the tube (40) from taking damage by the two compressing segments (53, 54) of the two opposing arms (51, 52).

8. The device of any of the preceding claims 5-7, wherein the two opposing arms (51, 52) of the spring clip (50) are connected.

9. The device of any of the preceding claims, wherein the spring clip (50) is made of a resilient material, such as metal or plastic material, wherein the spring clip (50) has its natural shape in the activated position (70) and wherein the spring clip (50) is urged to the outside in the deactivated position (80).

10. The device of any of the preceding claims, wherein the spring clip (50) is fabricated of a single piece of material.

11. The device of any of the preceding claims, wherein the flexible tube (40) is made of silicon.

12. The device of any of the preceding claims, wherein the spring clip (50) does not extend beyond the inner cavity (30) of the catheter hub (20).

13. The device of any of the preceding claims, wherein the spring clip (50) is in the activated position (70) before using the device (10) for a first time.

14. A flexible spring clip (50) for use in an intravenous catheter device (10) according to any of the preceding claims.

## Patentansprüche

1. Intravenöse Kathetervorrichtung (10) mit:
a. einem Katheteranschlussstück (20) mit einem inneren Hohlraum (30);
b. einem flexiblen Rohr (40), das innerhalb des inneren Hohlraums (30) angeordnet ist, um einen schließbaren Durchgang (90) für Fluid durch das Katheteranschlussstück (20) hindurch vorzusehen; und
c. einem flexiblen Federclip (50), der zwischen einer deaktivierten Position (80) und einer aktivierten Position (70) beweglich ist, wobei der Federclip (50) das flexible Rohr (40) in der aktivierten Position (70) komprimiert, um den Durchgang (90) zu schließen;
d. wobei der Federclip (50) von der aktivierten Position (70) zu der deaktivierten Position (80) beweglich ist, wenn ein Fluidverbinder (60) in das Katheteranschlussstück (20) eingesetzt wird,
**dadurch gekennzeichnet, dass**
der innere Hohlraum (30) des Katheteranschlussstücks (20) zwei Zurückhaltehohlräume (31, 32) aufweist, in denen wenigstens ein Teil des Federclips (50) angeordnet ist, wobei jeder Zurückhaltehohlraum (31, 32) eine Trapezform bei dem distalen Ende hat.

2. Vorrichtung nach Anspruch 1, wobei der Federclip (50) beweglich ist, ohne dass der Benutzer den Federclip berühren muss.

3. Vorrichtung nach einem der vorhergehenden Ansprüche, wobei der Federclip (50) von der deaktivierten Position (80) in die aktivierte Position (70) beweglich ist, wenn der Fluidverbinder (60) von dem Katheteranschlussstück (20) zurückgezogen wird.

4. Vorrichtung nach dem vorhergehenden Anspruch, wobei wenigstens ein Teil des Federclips (50) in den distalen Abschnitt von jedem der Zurückhaltehohlräume (31, 32) bewegt wird, wenn der Federclip (50) von aktivierten Position (70) in die deaktivierte Position (80) bewegt wird, wodurch bewirkt wird, dass der Federclip (50) das flexible Rohr (40) nicht länger komprimiert.

5. Vorrichtung nach einem der vorhergehenden Ansprüche, wobei der Federclip (50) zwei gegenüberliegende Arme (51, 52) aufweist.

6. Vorrichtung nach dem vorhergehenden Anspruch, wobei die zwei gegenüberliegenden Arme (51, 52) jeweils ein Segment (53, 54) aufweisen, das in einer Richtung zu dem Rohr (40) hin ausgebildet ist, und wobei jedes der zwei Segmente (53, 54) das Rohr (40) in der aktivierten Position (70) komprimiert.

7. Vorrichtung nach Anspruch 6, wobei die zwei Segmente (53, 54) des Weiteren ein gerundetes Ende (55, 56) aufweisen, um zu verhindern, dass das Rohr (40) durch die zwei komprimierenden Segmente (53, 54) der zwei gegenüberliegenden Arme (51, 52) beschädigt wird.

8. Vorrichtung nach einem der vorhergehenden Ansprüche 5 bis 7, wobei die zwei gegenüberliegenden Arme (51, 52) des Federclips (50) verbunden sind.

9. Vorrichtung nach einem der vorhergehenden Ansprüche, wobei der Federclip (50) aus einem elastischen Material, wie einem Metall oder einem Kunststoffmaterial, gemacht ist, wobei der Federclip (50) in der aktivierten Position (70) seine natürliche Form hat und wobei der Federclip (50) in der deaktivierten Position (80) zu der Außenseite gedrängt ist.

10. Vorrichtung nach einem der vorhergehenden Ansprüche, wobei der Federclip (50) aus einem einzelnen Materialstück hergestellt ist.

11. Vorrichtung nach einem der vorhergehenden Ansprüche, wobei das flexible Rohr (40) aus Silicium gemacht ist.

12. Vorrichtung nach einem der vorhergehenden Ansprüche, wobei der Federclip (50) sich nicht über den inneren Hohlraum (30) des Katheteranschlussstücks (20) hinaus erstreckt.

13. Vorrichtung nach einem der vorhergehenden Ansprüche, wobei der Federclip (50) in der aktivierten Position (70) ist, bevor die Vorrichtung (10) das erste Mal verwendet wird.

14. Flexibler Federclip (50) zur Verwendung in einer intravenösen Kathetervorrichtung (10) nach einem der vorhergehenden Ansprüche.

## Revendications

1. Dispositif de cathéter intraveineux (10), comprenant :
a. un moyeu de cathéter (20) comprenant une cavité interne (30) ;
b. un tube flexible (40) agencé à l'intérieur de la cavité interne (30) pour fournir un passage refermable (90) pour un fluide à travers le moyeu de cathéter (20) ; et
c. une pince à ressort flexible (50) qui est mobile entre une position désactivée (80) et une position activée (70), dans lequel la pince à ressort (50) comprime le tube flexible (40) dans la position activée (70) pour fermer le passage (90) ;
d. la pince à ressort (50) étant mobile de la position activée (70) à la position désactivée (80) lorsqu'un connecteur de fluide (60) est inséré dans le moyeu de cathéter (20),
**caractérisé en ce que** la cavité interne (30) du moyeu de cathéter (20) comprend deux cavités de retenue (31, 32) dans lesquelles au moins une partie de la pince à ressort (50) est agencée, dans lequel chaque cavité de retenue (31, 32) présente une forme trapézoïdale à l'extrémité distale.

2. Dispositif selon la revendication 1, dans lequel la pince à ressort (50) est mobile sans que l'opérateur n'ait à toucher la pince à ressort.

3. Dispositif selon l'une quelconque des revendications précédentes, dans lequel la pince à ressort (50) est mobile de la position désactivée (80) à la position activée (70) lorsque le connecté de fluide (60) est rétracté à partir du moyeu de cathéter (20).

4. Dispositif selon la revendication précédente, dans lequel au moins une partie de la pince à ressort (50) est déplacée dans la partie distale de chacune des cavités de retenue (31, 32) lorsque la pince à ressort (50) est déplacée de la position activée (70) à la position désactivée (80), en amenant la pince à ressort (50) à ne plus comprimer le tube flexible (40).

5. Dispositif selon l'une quelconque des revendications précédentes, dans lequel la pince à ressort (50) comprend deux bras opposés (51, 52).

6. Dispositif selon la revendication précédente, dans lequel les deux bras opposés (51, 52) comprennent chacun un segment (53, 54) formé en direction du tube (40), et dans lequel chacun des deux segments (53, 54) comprime le tube (40) dans la position activée (70).

7. Dispositif selon la revendication 6, dans lequel les deux segments (53, 54) comprennent en outre une extrémité arrondie (55, 56) pour empêcher le tube (40) de subir des dommages par les deux segments de compression (53, 54) des deux bras opposés (51, 52).

8. Dispositif selon l'une quelconque des revendications précédentes 5 à 7, dans lequel les deux bras opposés (51, 52) de la pince à ressort (50) sont reliés.

9. Dispositif selon l'une quelconque des revendications précédentes, dans lequel la pince à ressort (50) est réalisée en un matériau élastique, tel qu'un métal ou un matériau plastique, dans lequel la pince à ressort (50) présente sa forme naturelle dans la position activée (70) et dans lequel la pince à ressort (50) est poussée vers l'extérieur dans la position désactivée (80).

10. Dispositif selon l'une quelconque des revendications précédentes, dans lequel la pince à ressort (50) est fabriquée en une seule pièce de matériau.

11. Dispositif selon l'une quelconque des revendications précédentes, dans lequel le tube flexible (40) est réalisé en silicone.

12. Dispositif selon l'une quelconque des revendications précédentes, dans lequel la pince à ressort (50) ne s'étend pas au-delà de la cavité interne (30) du moyeu de cathéter (20).

13. Dispositif selon l'une quelconque des revendications précédentes, dans lequel la pince à ressort (50) est dans la position activée (70) avant d'utiliser le dispositif (10) pour une première fois.

14. Pince à ressort flexible (50) à utiliser dans un dispositif de cathéter intraveineux (10) selon l'une quelconque des revendications précédentes.
